Europäisches Patentamt

**European Patent Office**

Office européen des brevets

(19)

(11) Publication number: **0 028 929**
**B1**

## EUROPEAN PATENT SPECIFICATION

(12)

(45) Date of publication of patent specification: **02.05.84**

(51) Int. Cl.³: **A 61 J 7/00**

(21) Application number: **80303995.7**

(22) Date of filing: **07.11.80**

(54) Dose indicator for inhalers.

(30) Priority: **07.11.79 GB 7938513**

(43) Date of publication of application:
**20.05.81 Bulletin 81/20**

(45) Publication of the grant of the patent:
**02.05.84 Bulletin 84/18**

(84) Designated Contracting States:
**AT BE CH DE FR IT LI LU NL SE**

(56) References cited:
**WO - A - 80/00755**
**BE - A - 870 610**
**DE - A - 2 721 824**
**US - A - 4 034 757**

(73) Proprietor: **STERWIN AG.**
**Zeughausgasse 9**
**CH-6300 Zug (CH)**

(72) Inventor: **Poore, Albert Cyril Glanville**
**11 Woodside Road**
**New Malden Surrey (GB)**

(74) Representative: **Smith, Martin Stanley et al,**
**Stevens, Hewlett & Perkins 5, Quality Court**
**Chancery Lane**
**London WC2A 1HZ (GB)**

Courier Press, Leamington Spa, England.

## Dose indicator for inhalers

The invention relates to an indicator arrangement which is used in conjunction with inhalers for asthma relief. The drugs used for asthma relief are powerful and users are warned to restrict the dosage. However, it is sometimes difficult for a patient, a parent or a doctor to keep an accurate count of the number of doses taken over a particular period. The invention seeks to provide an automatic indicator for this purpose.

Proposals have been made hitherto for either (a) reminding a patient to take regular doses of a medicament or (b) recording the pattern of medicament usage. In the former category, publication WO—A—80/00755 describes a resettable alarm device which may be strapped to a medicament dispenser to give an alarm if no dose has been taken in a particular period. In the latter category U.S.—A—4034757 discloses an arrangement in which a medicament dispenser is contained in a housing and switches are arranged automatically to feed an electronic memory with information to record whether or not a dose has been taken in each of a number of successive hours.

Asthma sufferers take inhalter doses as required and not regularly. A reminder device is pointless. Also, while a simple recording device is useful for subsequent analysis of the pattern of usage, it cannot warn against a potential over-usage at the time a dose is required. The present invention provides a solution to this problem.

According to the invention there is provided an indicator unit, the unit comprising a case adapted to house a medicament dispenser, a timing circuit and a time switch mounted so as to be actuated each time the dispenser is used, and a battery, characterised in that the medicament dispenser is an asthma inhaler housed in the case in such a way that it can be used without removal from the case, the timing circuit is programmed with a predetermined dosage regimen, and there is provided indicator means, the timing circuit being responsive to operation of the switch and being effective to give an output to the indicator means which takes into account the usage of the inhaler in a time period preceding the current time, and the predetermined dosage regimen, so as to cause the indicator means to give a first indication if it is safe to take a current dose and a second indication if it is not safe to take a current dose.

Preferably the timer and indicator are such as to give a further indication if it is only moderately safe to take a current dose. Yet further indications can give the degree of safety of a current dose.

The time circuit may be composed of discrete circuit components and the required program may be determined by the values of specific components of the circuit. Alternatively, micro-computer techniques may be employed and the timer circuit may comprise a pre-programmed, and perhaps a re-programmable, micro-computer integrated-circuit.

The invention is applicable to the various kinds of inhalers which are available. For example, the inhaler may be of the type which dispenses a dispersal of liquid or dry powder. The drug may be dispersed and drawn in by sucking action or it may be contained in a pressurized aerosol dispenser or other means mechanical or electronic to atomise the drug. Aerosols may be actuated by manual depression of the spray head, by mechanical means of pushing the aerosol and spray head together, or by a pressure-responsive device which acts when the sufferer draws at the mouthpiece.

In inhalers which are actuated manually the timer switch is conveniently coupled to be operated by depression of the spray head. With pressure-actuated inhalers a pressure-responsive switch may be fitted at the mouthpiece.

The indicator means may be an audible indicator but preferably it is a visual display. The display may be digital, using light-emitting diodes (LED's) or liquid crystal devices (LCD's). Alternatively, the display may be in coded form using flashing and/or coloured lights conveniently LED's.

A difficulty with LED displays in particular is that there is a significant battery drain when the display is illuminated.

Thus, preferably the display is illuminated only on demand, perhaps by a manual switch but preferably automatically by a switch coupled to the lid.

It is preferable for the timer circuit to have a memory which allows the dosage to be totalled over, say, twenty-four hours. Furthermore, it is convenient if the memory allows totalisation of dosage over, say, 16 days. Preferably, therefore, the memory is non-volatile, so that should the battery be disconnected from the circuit between doses the information is not lost. With this arrangement the display switch becomes a battery switch which connects the display to the battery only when required.

The above-described facility for relatively long-term storage of dosage is useful for purposes of clinical analysis, for example for evaluation of the efficiency of a particular drug. The patient, the clinician and the doctor will have access to this information which can be LCD or LED. In the case of LED the doctor or clinician may connect a digital display device, or print-out device, which taps the long term memory and presents the information sequentially in 24 hours totals or in graphic form. In the case of LCD the information is immediately available on the unit in the same sequential manner by means of a switch.

The invention will further be described with reference to the accompanying drawings, of which

Figure 1 is a sectional elevation of an indicator unit according to one embodiment of the invention;

Figure 2 is a perspective view of the unit of Figure 1;

Figure 3 is a view showing the display of the unit of Figures 1 and 2;

Figure 4 is a perspective view of a second-form of the indicator unit according to the invention;

Figure 5 is a perspective view of the unit of Figure 4 ready for use;

Figure 6 is a sectional elevation of another indicator unit according to the invention;

Figure 7 is a perspective view of the unit of Figure 6; and

Figure 8 is a view showing the display of the unit of Figures 6 and 7.

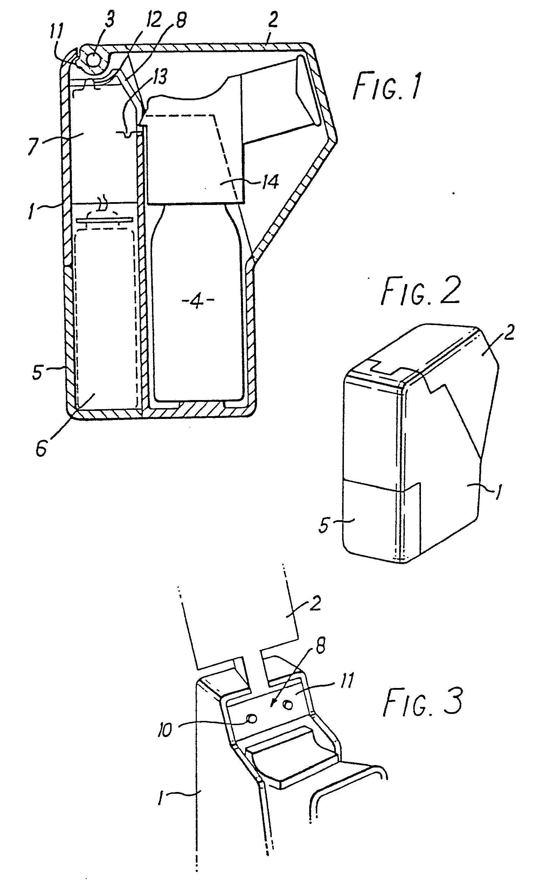

Referring to Figure 1 the indicator unit comprises a plastics casing having a body 1 with a lid 2 hinged at 3. The casing houses the drug container which is an aerosol 4 which may be removed and replaced when exhausted. The body 1 has a sliding cover 5 which allows a battery 6 to be inserted. A timer circuit 7 of discrete components is included and this operates a visual display 8 consisting of a red LED 9 and a green LED 10.

The lid 2 is provided with a detent 11 which, when the lid is opened, closes contacts of a microswitch 12. This allows the display to be connected to the timer circuit. Otherwise, the display is not connected. A timer microswitch 13 is positioned to be operated when the spray head 14 of the inhaler 4 is depressed to dispense a dose. The timer circuit registers the operation of the switch 13 and has memory which, in conjunction with an electronic timer, determines the number of doses dispensed in the recent past. The usual maximum safe dosage is generally considered to be three doses every three hours, although this may be determined by the drug manufacturer. The timer circuit is programmed to operate to show a limit signal within a predetermined dosage regimen. For example, for a dosage of three doses every three hours the indications would be in accordance with the following code:

If no doses have been taken in the last three hours, the green LED gives a sequence of three-flash bursts, showing that up to three doses may be taken. If one dose has been taken in the last three · hours, the green LED gives a sequence of two-flash bursts. If two doses have been taken in the last three hours the green LED gives a sequence of single spaced flashes. If three doses have been taken in the last three hours the red LED is illuminated, either continuously or in flashes, to indicate the inadvisability of taking further doses.

A further indication is given when the restriction is about to be released, say fifteen minutes beforehand. Then the green LED is illuminated in conjunction with the red LED, in alternate flashes.

The timer circuit has a memory which stores the number of doses taken in say 16 days. This can be tapped for digital indication by means of a plug and socket (not shown).

The unit of Figures 4 and 5 has the LED's 9 and 10 visible externally. The unit is made ready for use by sliding a catch 15 across in the direction of arrow 16. A cover 17 is then swivelled in the direction of arrow 18. This brings the inhaler nozzle to a position ready for use and switched on the LED display. The inhaler is actuated by squeezing a side portion 19 of the casing. This portion may be swung outwardly for battery replacement.

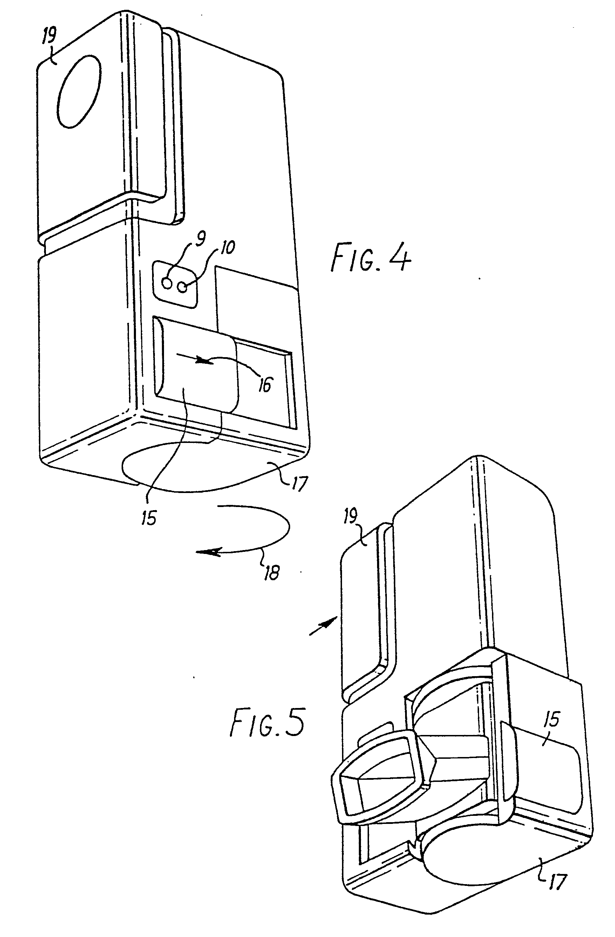

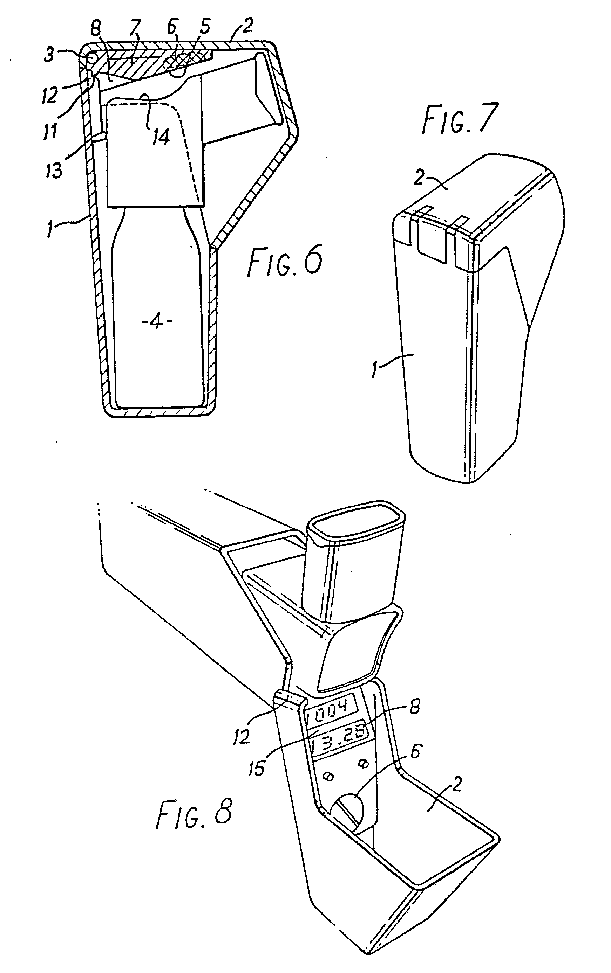

Referring to Figures 6, 7 and 8, the indicator unit comprises a case, timer circuit, switches and display etc., which have the same designations as in the embodiments of Figures 1 to 5. In the present embodiment, however, the visual display 8 consists of an LCD panel giving up to eight digit information with up to eight special symbols if required.

The maximum safe dosage is usually determined by the drug manufacturer, the LCD method however will provide digital or graphical information which can be related to precise instructions given by the doctor or clinician.

In this embodiment the timer circuit comprises a pre-programmed micro-computer integrated circuit. A large number of alternative digital, graphical, symbolic and audible messages can be provided giving precise information to the patient, doctor or clinician. For example and in accordance with the alternative LCD system the display panel could be given a two colour image in which case digital information on doses taken within the three hour period would be displayed on one colour section and doses taken over the normal three doses every 3 hour regimen would be displayed on the other different colour section. Alternatively information concerning doses taken over the recommended limit can be displayed digitally in a repeat form or series of flashes or by use of a flashing symbol. In all options it is possible to provide a perpetual clock and to indicate timing between doses or time when last dose was taken or when it is reasonable to take another dose. Digital or symbolic information would however preferably be quite simple for the patient to understand but the doctor or clinician may wish to have access to more detailed information concerning number of doses taken, sequence and so on with immediate daily totalisation and daily totals on a sequential basis. It is also possible to provide information by means of an audible electronic speaking voice. The LCD electronic method would provide immediate visual access to the memory without the need to plug in for an external digital display. The unit however could be produced with a socket from which, by means of a plug and lead, a print-out could be made of the information contained in the memory. This would be of particular value to the clinician.

The unit of Figure 8 has the LCD panel 15 visible when the mouthpiece or lid 2 cover is opened. The unit is then made ready for use by closing contacts of microswitch 12 and the display is switched on. In order to reduce consumption of power the LCD display will be automatically switched off after a predetermined period of time and can be reinstated by closing and re-opening the mouthpiece cover.

A suitable micro-processor for the timing circuit of the embodiment of Figures 6 to 8 is that marketed by ITT semiconductors under the designation SAA 6000. This is a mask programmable device which can be thus programmed with the dosage regimen to give any required form of output — including audible.

## Claims

1. An indicator unit, the unit comprising a case (1) adapted to house a medicament dispenser (4), a timing circuit (7), and a time switch (13) mounted so as to be actuated each time the dispenser (4) is used, and a battery (6), characterised in that the medicament dispenser is an asthma inhaler (4) housed in the case (1) in such a way that it can be used without removal from the case, the timing circuit (7) is programmed with a predetermined dosage regimen, and there is provided indicator means (8), the timing circuit (7) being responsive to operation of the switch (13) and being effective to give an output to the indicator means which takes into account the usage of the inhaler in a time period preceding the current time, and the predetermined dosage regimen, so as to cause the indicator means to give a first indication if it is safe to take a current dose and a second indication if it is not safe to take a current dose.

2. An indicator as claimed in claim 1 wherein the indicator means (8) gives a visual display in coded form using flashing and/or coloured lights (9, 10).

3. An indicator as claimed in claim 1 wherein the indicator means (8) gives a digital display.

4. An indicator as claimed in any of the preceding claims wherein the case (1) has a lid (2) which must be opened before the inhaler (4) can be used and a second switch (12) is positioned to be operated when the lid is opened, the indicator means (8) being operated to give a display by operation of the second switch (12).

5. An indicator unit as claimed in claim 4 wherein the timing circuit (7) includes means for extinguishing the display a predetermined time after the lid (2) has been opened.

6. An indicator unit as claimed in any of the preceding claims wherein the timer circuit (8) includes a memory which allows the dosage to be recorded over at least 24 hours.

7. An indicator unit as claimed in claim 6 wherein the memory allows the dosage to be recorded over at least 16 days.

8. An indicator as claimed in claim 7 wherein the memory is non-volatile.

## Patentansprüche

1. Indikatoreinheit bestehend aus einem Gehäuse (1) zur Aufnahme eines Medikament-Spenders (4), einem Zeitkreis (7) und einem Zeitschalter (13), der so montiert ist, daß er betätigt wird, jedesmal wenn der Spender (4) benutzt wird, und einer Batterie (6), dadurch gekennzeichnet, daß der Medikament-Spender ein Asthma-Inhalator (4) ist, der in dem Gehäuse (1) so angeordnet ist, daß er benutzt werden kann, ohne daß er aus dem Gehäuse entfernt wird, der Zeitkreis (7) mit einem vorbestimmten Dosierungsprogramm programmiert ist, und eine Anzeigeeinrichtung (8) vorgesehen ist, wobei der Zeitkreis (7) auf die Betätigung des Schalters (13) anspricht und effektiv ein Ausgangssignal an die Anzeigeeinrichtung gibt, die die Benutzung des Inhalators in einem Zeitabschnitt vor dem gegenwärtigen Zeitpunkt und das vorbestimmte Dosierungsprogramm berücksichtigt, um die Anzeigeeinrichtung zu veranlassen, eine erste Anzeige zu machen, wenn es risikolos ist, eine übliche Dosis zu nehmen, und eine zweite Anzeige, wenn es riskant ist, eine übliche Dosis zu nehmen.

2. Indikator nach Anspruch 1, bei dem die Anzeigeeinrichtung (8) eine visuelle Anzeige in kodierter Form bit blinkenden und/oder bunten Lichtern (9, 10) liefert.

3. Indikator nach Anspruch 1, bei dem die Anzeigeeinrichtung (8) eine digitale Anzeige liefert.

4. Indikator nach einem der vorhergehenden Ansprüche, bei dem das Gehäuse (1) einen Deckel (2) hat, der geöffnet werden muß, bevor der Inhalator (4) benutzt werden kann, und ein zweiter Schalter (12) so positioniert ist, daß er betätigt wird, wenn der Deckel geöffnet wird, wobei die Anzeigeeinrichtung (8) durch Betätigung des zweiten Schalters (12) betätigt wird, um eine Anzeige zu liefern.

5. Indikator nach Anspruch 4, bei dem der Zeitkreis (8) Einrichtungen aufweist, um die Anzeige nach einer vorbestimmten Zeit nach Öffnen des Deckels (2) zu löschen.

6. Indikator nach einem der vorhergehenden Ansprüche, bei dem der Zeitkreis (8) einen Speicher aufweist, mit dem die Dosierung über mindestens 24 Stunden aufgezeichnet werden kann.

7. Indikator nach Anspruch 6, bei dem der Speicher es ermöglicht, die Dosierung über mindestens 16 Tage aufzuzeichnen.

8. Indikator nach Anspruch 7, bei dem der Speicher nicht flüchtig ist.

## Revendications

1. Dispositif indicateur, comprenant un boîtier (1) apte à recevoir un distributeur de médicaments (4), un circuit de chronométrage (7) et un interrupteur de chronométrage (13) monté de façon à être actionné à chaque utilisation du distributeur (4), et une pile (6), caractérisé en ce que le distributeur de médicaments est un inhalateur pour l'asthme (6) disposé dans le boîtier (1) de façon à permettre son utilisation sans le retirer du boîtier, le circuit de chronométrage (7) est programmé selon un régime de dosage prédéterminé, et il est prévu des moyens indicateurs (8), le circuit de chronométrage (7) étant sensible à l'actionnement de l'interrupteur (13) et fournissant une sortie aux moyens indicateurs qui prend en compte les utilisations de l'inhalateur dans une période de temps précédent le temps courant, ainsi que le régime de dosage prédéterminé, de façon que les moyens indicateurs donnent une première indication s'il est sans danger de prendre une dose courante et une seconde indication s'il n'est pas sans danger de prendre une dose courante.

2. Indicateur selon la revendication 1, caractérisé en ce que les moyens indicateurs (8) fournissent un affichage visuel sous forme codée en utilisant des lumières clignotantes et/ou colorées (9, 10).

3. Indicateur selon la revendication 1, caractérisé en ce que les moyens indicateurs (8) fournissent un affichage numérique.

4. Indicateur selon l'une quelconque des revendications précédentes, caractérisé en ce que le boîtier (1) comprend un couvercle (2) qui doit être ouvert avant que l'inhalateur (4) puisse être utilisé et un second interrupteur (12) est positionné de façon à être actionné lorsqu'on ouvre le couvercle, les moyens indicateurs (8) étant actionnés pour fournir un affichage par actionnement du second interrupteur (12).

5. Dispositif indicateur selon la revendication 4, caractérisé en ce que le circuit de chronométrage (8) comprend des moyens d'extinction de l'affichage après un temps prédéterminé suivant l'ouverture du couvercle (2).

6. Dispositif indicateur selon l'une quelconque des revendications précédentes, caractérisé en ce que le circuit de chronométrage (8) comprend une mémoire qui permet la mise en mémoire du dosage sur au moins 24 heures.

7. Dispositif indicateur selon la revendication 6, caractérisé en ce que la mémoire permet la mise en mémoire du dosage sur au moins 16 jours.

8. Indicateur selon la revendication 7, caractérisé en ce que la mémoire est non volatile.

FIG. 1

FIG. 2

FIG. 3

*FIG. 4*

*FIG. 5*

FIG. 6

FIG. 7

FIG. 8